(19) Europäisches Patentamt / European Patent Office / Office européen des brevets

(11) **EP 2 660 245 A1**

(12) **EUROPEAN PATENT APPLICATION**
published in accordance with Art. 153(4) EPC

(43) Date of publication:
**06.11.2013 Bulletin 2013/45**

(21) Application number: **11853818.0**

(22) Date of filing: **01.11.2011**

(51) Int Cl.:
***C07F 9/53*** *(2006.01)* ***A61K 31/66*** *(2006.01)*
***A61P 25/28*** *(2006.01)*

(86) International application number:
**PCT/RU2011/000843**

(87) International publication number:
**WO 2012/091624 (05.07.2012 Gazette 2012/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2010 RU 2010153277**

(71) Applicant: **Federalnoe Gosudarstvennoe Bydzhetnoe Uchrezhdenie Nauki Institut Fiziologicheski Aktivnikh Veschestv Rossiiskoi Akademii Nauk (IFAV RAN)**
**Moskovskaya obl. 142432 (RU)**

(72) Inventors:
- **YARKEVICH, Alla Nikolaevna**
**Nogisnkiy raion**
**Moskovskaya obl. 142432 (RU)**
- **SAFRONOVA, Zoya Viktorovna**
**Nogisnkiy r-n**
**Moskovskaya obl. 142432 (RU)**
- **PETROVA, Ludmila Nikolaevna**
**Moskovskaya obl. 142432 (RU)**
- **BACHURIN, Sergey Olegovich**
**Moskovskaya obl. 142432 (RU)**
- **SEFIROV, Nikolay Serafimovich**
**Moscow 117421 (RU)**

(74) Representative: **King, Lawrence**
**A.A. Thornton & Co.**
**235 High Holborn**
**London WC1V 7LE (GB)**

(54) **PHYSIOLOGICALLY ACTIVE BIS-DIALKYLAMIDES OF PHOSPHORYL-SUBSTITUTED 1,4-DICARBOXYLIC ACIDS AND METHOD FOR PRODUCING SAME**

(57) To produce physiologically active bis-dialkylamides of phosphorylated 1,4-dicarboxylic acids of formula (I)

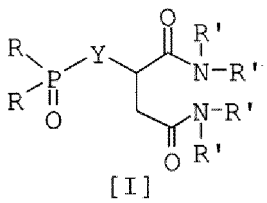

[I] ,

wherein each R can be straight or branched alkyl, unsubstituted or substituted phenyl, wherein substituents of the phenyl group can be from 1 to 5 halogen atoms, from 1 to 5 lower alkyl groups, from 1 to 5 lower alkoxy groups, from 1 to 5 dialkylamino groups; R' can be straight or branched alkyl; Y is a direct bond or a methylene group, a mixture of the corresponding phosphorylated 1,4-dicarboxylic acids is heated with hexaalkyl triamidophosphites of general formula $(R'_2N)_3P$ in an organic solvent within a temperature range from 20 to 80°C until the reaction is complete followed by separation and purification. The compounds of general formula (I) thereby produced possess neuroprotective properties and can be used in medicine.

EP 2 660 245 A1

## Description

The present invention relates to the field of organic chemistry and can be used in medicine as new physiologically active compounds, in particular, compounds with neuroprotective activity.

Synthesis of new compounds of substituted 1,4-diamides of dicarboxylic acids is an urgent task since many of them have different biological activities and can find application in medical practice [B.O. Koz'minykh. Synthesis and biological activity of substituted amides and hydrazides of 1,4-dicarboxylic acids (a review). Pharmaceutical Chemistry Journal (Rus). 2006, v.40, No 1, pp.9-17]. Amides and diamides of unsaturated 1,4-dicarboxylic acids and succinic acid and its homologs are most thoroughly studied.

It is known that dialkylamides of $\alpha,\omega$-dicarboxylic acids can be produced from the corresponding monoamides, CO and amines [*M.C. Cesa, R.A. Dubbert, J.D. Burrington. 1,6-Hexanediamides from 3-pentenamides.* EP 186,950]. Alkyl-substituted diamides of succinic acid which are the closest to the claimed compounds were produced by the same method [*J. Kadelka, H.-H. Schwarz, Process for the preparation of substituted succinic acid amides.* US Pat. 4,588,833].

Unsubstituted diamides of succinic acid were also produced from a corresponding diester of succinic acid and amines in alcohol in the presence of alkali metal alcoholates [*V.P. Kucesky. Method of making amides of dimethylamine and piperazine.* US Pat 3,288,794].

An effect of heteroatom substituents in methylene moieties on the biological activity of a molecule has not been studied in practice since methods of synthesizing such compounds are developed poorly.

There is known a method of producing only one compound from the claimed group of bis(dialkylamides) of phosphoryl-
substituted 1,4-dicarboxylic acids, namely 1,4-bis(dibutylamide) of 2-(diphenylphosphinoyl)succinic acid *[*A.N. Turanov, V.K. Karandashev, A.N. Yarkevich, Z. V. Safronova, A.V. Kharitonov, N.I. Rodygina, A.M. Fedoseev. Extraction of U (VI), Th(IV), Pu(IV), Am(III), and rare-earth elements from nitric acid solutions with diphenyl(dialkylcarbamoylmethyl) phosphine oxides substituted in the methylene bridge. Radiochemistry, 2004, v.46, issue 5, pp.427-432]. It has been found that the resulting compound exhibits extraction properties.

The method for producing the above compounds considered as a prototype comprises the following steps: adding dropwise a solution of hexabutyl triamidophosphite $(Bu_2N)_3P$ to a suspension of 2-diphenylphosphinoyl-succinic acid in toluene followed by heating the reaction mixture for 2 hours under boiling toluene, i.e. at 110°C. The resulting mixture of compounds is to be separated by column chromatography to obtain the target compound with a yield of about 40%.

An objective of the present invention is, on the one hand, to expand an arsenal of agents with physiological activity. On the other hand, an equally important objective is to develop a method for their synthesis, which method provides such compounds with a high yield.

The objective is addressed by finding a group of bis(dialkylamides) of phosphoryl-substituted 1,4-dicarboxylic acids of general formula (I)

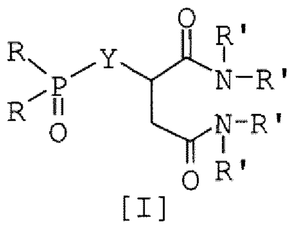

[I] ,

wherein each R can be straight or branched lower alkyl, unsubstituted or substituted phenyl, wherein the substituents of the phenyl group are selected from: from 1 to 5 halogen atoms, from 1 to 5 lower alkyl groups, from 1 to 5 lower alkoxy groups, from 1 to 5 dialkylamino groups; Y is a direct bond or a methylene group; R' can be straight or branched lower alkyl; having neuroprotective properties.

The inventors have surprisingly found that the inventive compounds of general formula (I), which contain a broad set of stable phosphine oxide moieties, can find application in medicine as neuroprotective compounds due to new properties thereof that cannot be derived from their chemical structure.

The most preferable compounds of formula (I) are the following:

$N^1$,$N^1$,$N^4$,$N^4$-tetrabutyldiamide of 2-(diphenylphosphinoyl)succinic acid;
$N^1$,$N^1$,$N^4$,$N^4$-tetrabutyldiamide of 2- [(2-methylphenyl)-phosphinoyl]succinic acid;
$N^1$,$N^1$,$N^4$,$N^4$-tetrabutyldiamide of 2-[bis(2,5-dimethylphenyl)-phosphinoyl]succinic acid;
$N^1$,$N^1$,$N^4$,$N^4$-tetrabutyldiamide of 2-[bis(4-dimethylaminophenyl)-phosphinoyl]succinic acid;

$N^1,N^1,N^4,N^4$-tetrabutyldiamide of 2-[bis(4-chlorophenyl)-phosphinoyl]succinic acid;
$N^1,N^1,N^4,N^4$-tetrabutyldiamide of 2-[bis(4-methoxyphenyl)-phosphinoyl]succinic acid;
$N^1,N^1,N^4,N^4$-tetrabutyldiamide of 2-(di-n-butylphosphinoyl)-succinic acid;
$N^1,N^1,N^4,N^4$-tetrabutyldiamide of 2-[bis(4-methoxyphenyl)-phosphinoylmethyl]succinic acid;
$N^1,N^1,N^4,N^4$-tetrabutyldiamide of 2-[bis(2,5-dimethylphenyl)-phosphinoylmethyl]succinic acid;
$N^1,N^1,N^4,N^4$-tetraethyldiamide of 2-(diphenylphosphinoylmetyl)succinic acid.

Another aspect of the invention is a method for producing bis-dialkylamides of phosphoryl-substituted 1,4-dicarboxylic acids of general formula (I) to obtain the target compounds with a yield of up to 75-95%.

The proposed method differs in the following points. The applicant has found that when the reaction is conducted under temperature of from 20 to 80°C in organic solvents selected from the group consisting of aromatic hydrocarbons or saturated hydrocarbons, only the target products, rather than mixtures of compounds, are produced with high yields because the reaction is not complicated by undesired processes of decarboxylation at this range of temperature, and proceeds, however, with a sufficiently high rate.

This method is universal and can be used for the synthesis of all claimed compounds (Scheme 1):

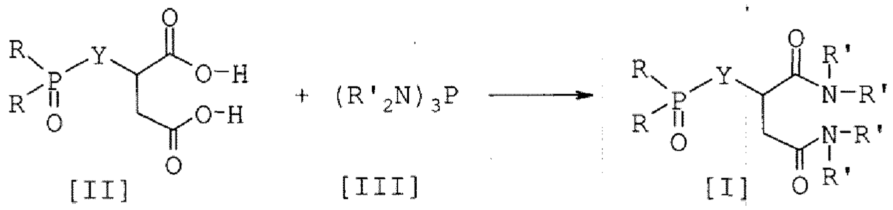
[II] [III] [I]

Scheme 1

Methods for synthesizing initial phosphoryl-substituted 1,4-dicarboxylic acids of general formula (II) have been developed by the applicant and are currently available [*A.N. Yarkevich, Z.V. Safronova, A.N. Pushin, S.O. Bachurin, N.S. Zefirov. A method for producing phosphorylated 1,4-dicarboxylic acids.* RU patent 2405789 according to application 2008129151*. A.N. Yarkevich, Z.V. Safronova, A.N. Pushin*, *S.O. Bachurin, N.S. Zefirov. A process for preparing salts of phosphorylated 1,4-dicarboxylic acids.* RU patent 2413733 according to application 2008129150.].

Hexaalkyl triamidophosphites $(R'_2N)_3P$ are also known compounds which are produced in an industrial scale from available raw materials.

A general method for synthesizing bis(dialkylamides) of phosphoryl-substituted 1,4-dicarboxylic acids of formula (I) is as follows.

Hexaalkyl triamidophosphites $(R'_2N)_3P(III)$ taken in a calculated or excess amount is added to a solution/ suspension of a phosphoryl-substituted 1,4-dicarboxylic acid in an anhydrous solvent, and the reaction mixture is stirred at temperature of from 20 to 80°C until the reaction is complete. The organic solution is washed with water, dried with $Na_2SO_4$, and the solvent is removed under vacuum. Before biological testing, the resulting oily product is additionally purified by column chromatography on silica gel L 100/160 $\mu$m (eluent is chloroform or a mixture of benzene-acetone (3:1)), after that in most cases the compounds are crystallized. The synthesized compounds are stable oily or crystalline compounds which are readily soluble in most organic solvents.

The following examples illustrate the claimed method of synthesis and features of the specific compounds obtained by the claimed method, without any limitations of the application of the present invention.

**Example 1.** $N^1,N^1,N^4,N^4$-tetrabutyldiamide of 2-(diphenylphosphinoyl)succinic acid $[C_6H_5]_2P(O)CH(CH_2CONBu_2)CONBu_2$ (Compound 1)

A solution of hexabutyl triamidophosphite $(Bu_2N)_3P$ in an amount of 1.08 g (0.0026 mol) in 2 ml of anhydrous benzene was added dropwise to 0.96 g (0.0030 mol) of a suspension of 2-(diphenylphosphinoyl)succinic acid in 15 ml of anhydrous benzene, and the mixture was heated for 1 hour at gentle boiling of the solvent. The reaction mixture was washed with water (3 x 7 ml), dried with $Na_2SO_4$, and the solvent was removed under vacuum. The compound (1) in an amount of 1.45 g was obtained as oil with a yield of 90%. The product was purified by column chromatography on silica gel L 100/160 $\mu$m. 1.30 g (80%) of $N^1,N^1,N^4,N^4$-tetrabutyldiamide 2-(diphenylphosphinoyl)succinic acid were eluted with chloroform as an oil. $^1H$ NMR ($CDCl_3$, $\delta$, ppm): 0.84 m (12H, $4CH_3$); 1.20 m (8H, $4CCH_2CCN$); 1.36 m (8H, $4CCCH_2CN$); 2.60 m, 1H and 3.00 m, 1H ($CH_2CP$); 3.16 m (8H, $2CH_2NCH_2$); 4.32 m (1H, CHP); 7.50 m, 6H, 7.86 m, 2H and 8.06 m, 2H($2C_6H_5$). $^{31}P$ NMR ($CDCl_3$, $\delta$, ppm): 31.97.

**Example 2.** $N^1,N^1,N^4,N^4$-tetrabutyldiamide of 2-[(2-methylphenyl)-phosphinoyl]succinic acid $[2\text{-}CH_3C_6H_4]_2P(O)CH(CH_2CONBu_2)CONBu_2$ (Compound 2)

The compound was synthesized similarly to compound (1) from $(Bu_2N)_3P$ (0.83 g, 0.002 mol) and 2-[(2-methylphenyl)phosphinoyl]succinic acid (1.04 g, 0.003 mol). Compound (2) was obtained in an amount of 1.55 g (91% yield) as oil. $^1H$ NMR ($CDCl_3$, δ, ppm): 0.94 m (12H, $4CH_3$); 1.28 m (8H, $4CCH_2CCN$); 1.44 m (8H, $4CCCH_2CN$); 2.48 s, 3H and 2.56 s 3H ($2CH_3$-Ar); 3.04 m (4H, $CH_2NCH_2$) and 3.40 m (4H, $CH_2NCH_2$); 3.18 m, 1H and 3.62 m, 1H ($CH_2CP$) ; 4.72 m (1H, CHP); 7.12 m, 1H, 7.10-7.90 m (8H, $2C_6H_4$). $^{31}P$ NMR ($CDCl_3$, δ, ppm): 38.96.

**Example 3.** $N^1,N^1,N^4,N^4$-tetrabutyldiamide of 2-[bis(2,5-dimethylphenyl)-phosphinoyl]succinic acid $[2,5\text{-}(CH_3)_2C_6H_3]_2P(O)\ CH\ (CH_2CONBu_2)\ CONBu_2$ (Compound 3)

The compound was synthesized similarly to compound (1) from $(Bu_2N)_3P$ (0.83 g, 0.002 mol) and 2-[bis(2,5-dimethylphenyl)-phosphinoyl]succinic acid (0.86 g, 0.0023 mol). Compound (3) was obtained in an amount of 1.30 g (95% yield) as oil. $^1H$ NMR ($CDCl_3$, δ, ppm): 0.82 m (12H, $4CH_3$); 1.20 m (8H, $4CCH_2CCN$); 1.36 m (8H, $4CCCH_2CN$); 2.22 s, 3H, 2.30 s, 6H and 2.38 s, 3H ($4CH_3$-Ar); 2.50 m, 1H and 3.50 m, 1H ($CH_2CP$), 2.96 m (4H, $CH_2NCH_2$) and 3.32 m (4H, $CH_2NCH_2$); 4.65 m (1H, CHP); 7.10-7.60 m (6H, $2C_6H_3$). $^{31}P$ NMR ($CDCl_3$, δ, ppm): 38.05.

**Example 4.** $N^1,N^1,N^4,N^4$-tetrabutyldiamide of 2-[bis(4-dimethylaminophenyl)-phosphinoyl]succinic acid $(4\text{-}Me_2NC_6H_4)_2P(O)CH(CH_2CONBu_2)CONBu_2$ (Compound 4)

The compound was synthesized similarly to compound (1) from $(Bu_2N)_3P$ (0.83 g, 0.002 mol) and 2-[bis(4-dimethylaminophenyl)-phosphinoyl]succinic acid (0.97 g, 0.0024 mol). Compound (4) was obtained in an amount of 1.25 g (83% yield, m.p. 92°C). $^1H$ NMR ($CDCl_3$, δ, ppm): 0.87 m (12H, $4CH_3$); 1.25 m (8H, $4CCH_2CC$); 1.50 m (8H, $4CCCH_2C$); 2.60 m, 1H and 2.90 m, 1H ($CH_2CP$); 3.00 s, (12H, $2CH_3NCH_3$); 3.20 m (8H, $2CH_2NCH_2$); 4.24 m (1H, CHP); 6.70 m, 4H and 7.72 m, 4H ($2C_6H_4$). $^{31}P$ NMR ($CDCl_3$, δ, ppm): 33.44.

**Example 5.** $N^1,N^1,N^4,N^4$-tetrabutyldiamide of 2-[bis(4-chlorophenyl)-phosphinoyl]succinic acid $(4\text{-}ClC_6H_4)_2P(O)CH(CH_2CONBu_2)CONBu_2$ (Compound 5)

The compound was synthesized similarly to compound (1) from $(Bu_2N)_3P$ (0.83 g, 0.002 mol) and 2-[bis(4-chlorophenyl)-phosphinoyl]succinic acid (0.97 g, 0.0025 mol). Compound (5) was obtained in an amount of 1.40 g (92% yield, m.p. 60°C). $^1H$ NMR ($CDCl_3$, δ, ppm): 0.86 m (12H, $4CH_3$); 1.20 m (8H, $4CCH_2CCN$); 1.40 m (8H, $4CCCH_2CN$); 2. 65 m, 1H and 2.95 m, 1H ($CH_2CP$), 3.08 m (4H, $CH_2NCH_2$); 3.26 m (4H, $CH_2NCH_2$); 4.30 m (1H, CHP); 7.45 m, 4H, 7.78 m, 2H and 8.10 m, 2H ($2C_6H_4$). $^{31}P$ NMR ($CDCl_3$, δ, ppm) : 30.73.

**Example 6.** $N^1,N^1,N^4,N^4$-tetrabutyldiamide of 2-[bis(4-methoxyphenyl)-phosphinoyl]succinic acid $[4\text{-}CH_3OC_6H_4]_2P(O)CH(CH_2CONBu_2)CONBu_2$ (Compound 6)

The compound was synthesized similarly to compound (1) from $(Bu_2N)_3P$ (0.83 g, 0.002 mol) and 2-[bis(4-methoxyphenyl)phosphinoyl]succinic acid (0.95 g, 0.0025 mol). Compound (6) was obtained in an amount of 1.21 g (81% yield) as oil. $^1H$ NMR ($CDCl_3$, δ, ppm): 0.83 m (12H, $4CH_3$) ; 1.18 m (8H, $2CCH_2CH_2C$); 1.32 m (8H, $2CCH_2CH_2C$); 2.58 m, 1H and 3.12 m, 1H (2H, $CH_2P$); 3.18 m (8H, $2CH_2NCH_2$); 3.85 m (6H, $2CH_3O$); 4.26 m (1H, CHP); 6.95 m, 4H, 7.75 m, 2H and 7.96 m, 2H ($2C_6H_4$). $^{31}P$ NMR ($CDCl_3$, δ, ppm): 31.96.

**Example 7.** $N^1,N^1,N^4,N^4$-tetrabutyldiamide of 2-(di-n-butylphosphinoyl)succinic acid $(n\text{-}C_4H_9)_2P(O)CH(CH_2CONBu_2)CONBu_2$ (Compound 7)

The compound was synthesized similarly to compound (1) from $(Bu_2N)_3P$ (0.43 g, 0.0012 mol) and 2-(di-n-butyl-phosphinoyl)-succinic acid (0.33 g, 0.0012 mol). Compound (7) was obtained in an amount of 0.59 g (98% yield, m.p. 46°C). $^1H$ NMR ($CDCl_3$, δ, ppm): 0.80 m (18H, $6CH_3$); 1.20-1.80 m (28H, $6CCH_2CH_2CN+CH_2PCH_2$); 2.50 m, 1H and 4.00 m, 1H ($CH_2CP$), 3.10-3.60 m (9H, $2CH_2NCH_2$+CHP); $^{31}P$ NMR ($CDCl_3$, δ, ppm): 50.60.

**Example 8.** $N^1,N^1,N^4,N^4$-tetrabutyldiamide of 2-[bis(4-methoxyphenyl)-phosphinoylmethyl]succinic acid $[4\text{-}CH_3OC_6H_4]_2P(O)CH_2CH(CH_2CONBu_2)CONBu_2$ Compound (8)

The compound was synthesized similarly to compound (1) from $(Bu_2N)_3P$ (0.83 g, 0.002 mol) and 2-[bis(4-methoxyphenyl)-phosphinoylmethyl]succinic acid (1.18 g, 0.003 mol). Compound (8) was obtained in an amount of 1.70

g (92% yield, m.p. 65°C). $^1H$ NMR ($CDCl_3$, δ, ppm): 0.85 m (12H, $4CH_3$); 1.00-1.58 m (16H, $4CCH_2CH_2C$); 2.24-2.90 m (4H, $CH_2P+CH_2CO$); 3.20 m (8H, $2CH_2NCH_2$); 3.56 m (1H, CHCP); 3.80 (6H, $2CH_3O$); 6.90 m, 4H and 7.68 m, 4H ($2C_6H_4$). $^{31}P$ NMR ($CDCl_3$, δ, ppm): 31.38.

**Example 9.** $N^1,N^1,N^4,N^4$-tetrabutyldiamide of 2-[bis(2,5-dimethylphenyl)-phosphinoylmethyl]succinic acid $[2,5-(CH_3)_2C_6H_3]_2P(O)CH_2CH(CH_2CONBu_2)CONBu_2$ (compound 9).

The compound was synthesized similarly to compound (1) from $(Bu_2N)_3P$ (0.83 g, 0.002 mol) and 2-[bis(2,5-dimethylphenyl)-phosphinoylmethyl]succinic acid (0.78 g, 0.002 mol). Compound (9) was obtained in an amount of 1.04 g (85% yield) as oil. $^1H$ NMR ($CDCl_3$, δ, ppm): 0.85 m (12H, $4CH_3$) ; 1.28 m (8H, $4CCH_2CCN$); 1.42 m (8H, $4CCCH_2CN$); 2.20 s, 2.22 s, 2.34 s and 2.36 s, 12H ($4CH_3$-Ar); 2.60-3.00 m (4H, $CH_2CCH_2$); 3.18 m (4H, $CH_2NCH_2$); 3.58 m (1H, CHCP); 7.08 m 2H, 7.22 m, 2H and 7.68 m, 2H (6H, $2C_6H_3$). $^{31}P$ NMR ($CDCl_3$, δ, ppm): 32.28.

**Example 10.** $N^1,N^1,N^4,N^4$-tetraethyldiamide of 2-(diphenylphosphinoylmetyl)succinic acid $[C_6H_5]_2P(O)CH_2CH(CH_2CONEt_2)CONEt_2$ (Compound 10)

The compound was synthesized similarly to compound (1) from $(Et_2N)_3P$ (0.49 g, 0.002 mol) and 2-(diphenylphosphinoylmetyl)succinic acid (0.65 g, 0.002 mol). $N^1,^N1,N^4,N^4$-tetraethyldiamide 2-(diphenylphosphinoylmetyl)succinic acid (10) was obtained in an amount of 0.81 g (91% yield, m.p. 116°C). $^1H$ NMR ($CDCl_3$, δ, ppm): 0.90-1.10 m (12H, $4CH_3$) ; 2.40-2.70 m and 2.85 m (4H, $CH_2P+CH_2CO$); 3.06-3.42 m (8H, $2CH_2NCH_2$); 3.60 m (1H, CHCP); 7.52 m, 6H and 7.80 m, 4H ($2C_6H_4$). $^{31}P$ NMR ($CDCl_3$, δ, ppm): 31.08.

**Study of physiological activity of the claimed compounds based on manifestation of their neuroprotective activity**

Neuroprotective action of the synthesized compounds was studied in $P_2$-fraction of the rat brain cortex synaptosomes as an *in vitro* convenient model for evaluation of the ability of various compounds to influence calcium uptake into the nerve endings. In the present invention, the applicant used the method of detecting the uptake of $^{45}Ca^{2+}$ into rat cortical synaptosomes when stimulated with glutamic acid.

Synaptosomes were obtained from the cerebral cortex of newborn Wistar rats (9-10 days) according to Hayosh's standard procedure [Hajos F.// Brain Res., 1975, Vol.93, No 3, pp. 485-489]. The brain was homogenized (glass-teflon) in 10 volumes of 0.32 M chilled sucrose (900 rpm). The homogenate was centrifuged for 10 minutes at 1500 g. The resulting supernatant was centrifuged for 20 minutes at 10000 g. To accumulate the radioactive label, the $P_2$-synaptosomal fraction was suspended in incubation buffer A (132 mM NaCl, 5 mM KCl, 5 mM HEPES, 10 mM glucose, pH 7.4 (protein concentration of 1.5-2 mg/ml)). The final concentration of $Ca^{2+}$ was 1.25 mM (1.4 μCi/ml). 200 mM glutamate was used to stimulate the uptake of $^{45}Ca^{2+}$ in synaptosomes. After 5 minutes of the incubation with glutamate at 37°C, the uptake of $^{45}Ca^{2+}$ was stopped by filtration through GF/B filters (Whatman, England), washing three times with cold buffer B (145 mM KCl, 10 mM Tris, 5.4 mM Trilon B, pH 7.4) [L.N. Petrova, S.O. Bachurin // Bull. Exp. Biol. Med., 2006., Vol. 142, No 7, pp.51-54]. The samples were analyzed by liquid scintillation beta analyzer (Russia). All tests were performed in three parallel replications in 2-3 independent experiments. The amount of $^{45}Ca^{2+}$ uptaced into synaptosomes was determined by the difference in the content of the label in the glutamate-induced uptake into synaptosomes and uptake into synaptosomes without agonist, and expressed as percentages of the control (control = 100%).

Specific uptake of $^{45}Ca^{2+}$ was calculated according to the following formula:

$$K_{(43/21)}=[(Ca_4 - Ca_3)/(Ca_2 - Ca_1)]\cdot 100\%,$$

wherein

$Ca_1$ is uptake of $^{45}Ca^{2+}$ in the control (without glutamate and a test compound),

$Ca_2$ is uptake of $^{45}Ca^{2+}$ in the presence of glutamate,

$Ca_3$ is uptake of $^{45}Ca^{2+}$ in the presence of a test compound (without glutamate),

$Ca_4$ is uptake of $^{45}Ca^{2+}$ in the presence of glutamate and a test compound. Statistical analysis was performed according to Student's test.

Testing of the compounds was performed at a concentration of 100 μM. Compounds 1-9 which have an inhibitory effect on the $^{45}Ca^{2+}$ uptake into rat cortical synaptosomes were examined in detail at four different concentrations to determine the $IC_{50}$ value (concentration of a compound at which 50% inhibition of calcium uptake takes place).

Table 1 presents data of studying the glutamate-induced uptake of $^{45}Ca^{2+}$ in rat cortical synaptosomes in the

presence of the claimed bis(dialkylamides) of phosphorylated 1,4-dicarboxylic acids (Compound 1-10) and the closest structural analogues of the claimed compounds, in particular phosphorylated 1,4 dicarboxylic acids and their esters (Compounds 11-15), as well as monobasic analogues, in particular phosphorylated amides of monobasic acetic acid (acetamides 16-18).

As can be seen from the table, in the studied concentrations the representatives of the claimed compounds (compounds 1-10) inhibit completely or significantly the calcium uptake into the synaptosomes of rat brain when stimulated with glutamate, which confirms their exceptional neuroprotective properties.

$IC_{50}$ values calculated for compounds 1-10 also confirm their high neuroprotective activity.

$LD_{50}$ values > 100 mg/kg determined for compounds 1-10 according to the standard method indicate that these compounds may be regarded as low-toxic ones.

In contrast to the claimed compounds (1-10), studies of their analogs show only a slight change in the level of the calcium uptake compared with that in the control (Compounds 11-18).

Thus, the studies confirm that the inventors have found that the claimed group of compounds exhibits new unexpected features that cannot be derived from the chemical structure of the compounds and their previously known features.

**Table 1**

| Studying of the glutamate-induced $^{45}Ca^{2+}$ uptake into rat cortical synaptosomes in the presence of bis(dialkylamides) of phosphorylated 1,4-dicarboxylic acids (Compound 1-10) and their analogues (Compounds 11-18) | | | | |
|---|---|---|---|---|
| No | Compound | Structure | $^{45}Ca^{2+}$ uptake (% vs. control) | $IC_{50}$, μM |
| 1 | $N^1,N^1,N^4,N^4$-Tetrabutyldiamide of 2-(diphenylphosphinoyl)succinic acid | 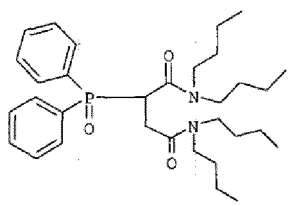 | 0 | 14.1 |
| 2 | $N^1,N^1,N^4,N^4$-Tetrabutyldiamide of 2-[(2-methylphenyl)-phosphinoyl] succinic acid | 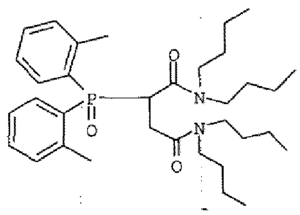 | 6.6±3.3 | 9.3 |
| 3 | $N^1,N^1,N^4,N^4$-Tetrabutyldiamide of 2-[bis(2,5-dimethylphenyl)-phosphinoyl]succinic acid | 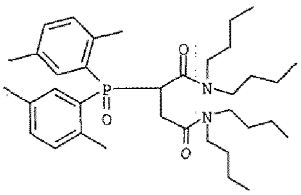 | 7.7±6.4 | 18.6 |
| 4 | $N^1,N^1,N^4,N^4$-Tetrabutyldiamide of 2-[bis(4-dimethylaminophenyl)-phosphinoyl] succinic acid | 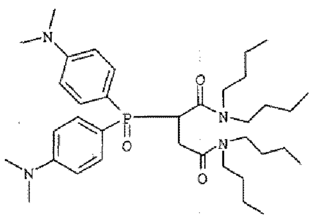 | 0 | 7.9 |
| 5 | $N^1,N^1,N^4,N^4$-Tetrabutyldiamide of 2-[bis(4-chlorophenyl)-phosphinoyl] succinic acid | 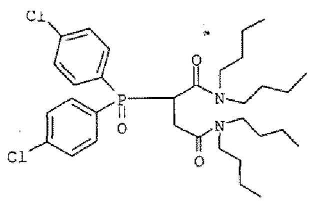 | 11.7±7.0 | 4.8 |

(continued)

| Studying of the glutamate-induced $^{45}Ca^{2+}$ uptake into rat cortical synaptosomes in the presence of bis(dialkylamides) of phosphorylated 1,4-dicarboxylic acids (Compound 1-10) and their analogues (Compounds 11-18) | | | | |
|---|---|---|---|---|
| No | Compound | Structure | $^{45}Ca^{2+}$ uptake (% vs. control) | $IC_{50}$, μM |
| 6 | $N^1,N^1,N^4,N^4$-Tetrabutyldiamide of 2-[bis(4-methoxyphenyl)-phosphinoyl] succinic acid | 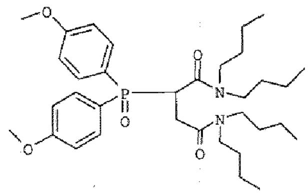 | 7.2±1.3 | 17.0 |
| 7 | $N^1,N^1,N^4,N^4$-Tetrabutyldiamide of 2-(di-n-butylphosphinoyl)succinic acid | 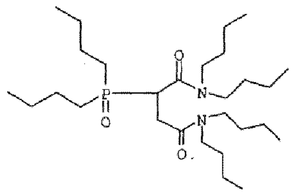 | 0 | 44.7 |
| 8 | $N^1,N^1,N^4,N^4$-Tetrabutyldiamide of 2-[bis(4-methoxyphenyl)-phosphinoylmethyl] succinic acid | 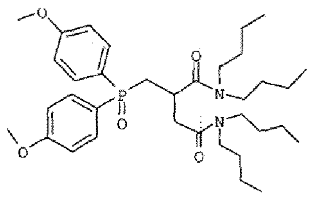 | 0 | 15.5 |
| 9 | $N^1,N^1,N^4,N^4$-Tetrabutyldiamide of 2-[bis(2,5-dimethylphenyl)-phosphinoylmethyl] succinic acid | 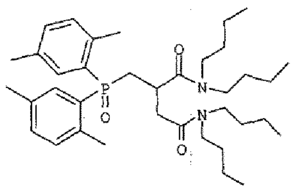 | 4.9±4.9 | 5.1 |
| 10 | $N^1,N^1,N^4,N^4$-Tetraethyldiamide of 2-(diphenylphosphinoylmetyl)succinic acid | 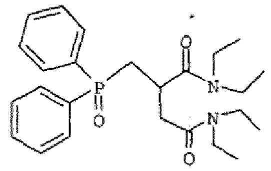 | 63.8±12.0 | |
| 11 | 2-[bis-(4-Dimethylaminophenyl)-phosphinoyl] succinic acid | 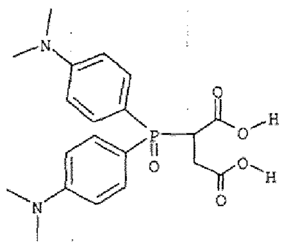 | 117.1±3.9 | |
| 12 | 2-[bis-(2,5-Dimethylphenyl)-phosphinoyl]succinic acid | 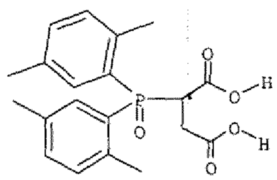 | 97.9±11.4 | |

(continued)

| Studying of the glutamate-induced $^{45}Ca^{2+}$ uptake into rat cortical synaptosomes in the presence of bis(dialkylamides) of phosphorylated 1,4-dicarboxylic acids (Compound 1-10) and their analogues (Compounds 11-18) | | | | |
|---|---|---|---|---|
| No | Compound | Structure | $^{45}Ca^{2+}$ uptake (% vs. control) | $IC_{50}$, µM |
| 13 | 2-(Diethylphosphinoyl)succinic acid | 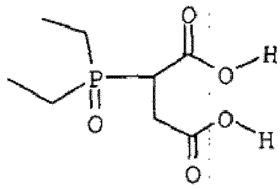 | 120.1±17.3 | |
| 14 | 2-[bis-(2,5-Dimethylphenyl)-phosphinoylmethyl] succinic acid | 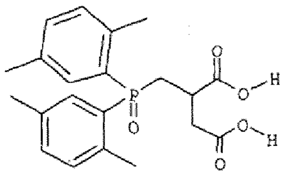 | 91±3.1 | |
| 15 | 2-(Diphenylphosphinoyl)succinic acid ethyl ester | 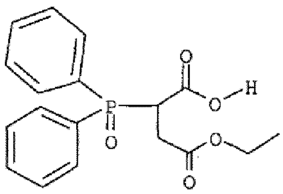 | 104±0.5 | |
| 16 | N,N-Dibutylamide of 2-(diphenylphosphinoyl)acetic acid | 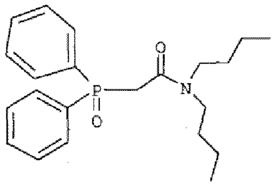 | 96.2±2.9 | |
| 17 | N,N-Diethylamide of 2-(diphenylphosphinoyl)acetic acid | 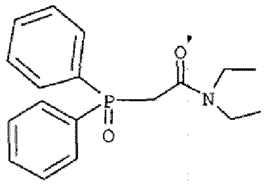 | 95.4+2.1 | |
| 18 | N,N-Diethylamide of 2-(diphenylphosphinoyl)-3-(diethylamino)propionic acid | 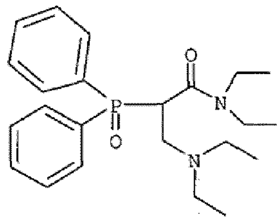 | 97.4+0.2 | |

## Claims

**1.** Compounds with neuroprotective activity which are bis-dialkylamides of phosphorylated 1,4-dicarboxylic acids of general formula (I),

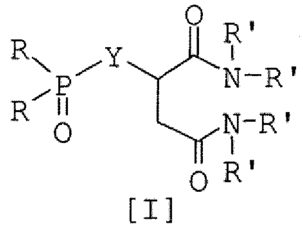

[I]

,

wherein each R can be straight or branched lower alkyl, unsubstituted or substituted phenyl, wherein substituents of the phenyl group can be from 1 to 5 halogen atoms, from 1 to 5 lower alkyl groups, from 1 to 5 lower alkoxy groups, from 1 to 5 dialkylamino groups; Y is a direct bond or a methylene group, R' can be straight or branched lower alkyl.

2. The compound according to claim 1, wherein the compound is $N^1,N^1,N^4,N^4$-tetrabutyldiamide of 2-(diphenylphosphinoyl)succinic acid.

3. The compound according to claim 1, wherein the compound is $N^1,N^1,N^4,N^4$-tetrabutyldiamide of 2-[(2-methylphenyl)-phosphinoyl]succinic acid.

4. The compound according to claim 1, wherein the compound is $N^1,N^1,N^4,N^4$-tetrabutyldiamide of 2-[bis(2,5-dimethylphenyl)-phosphinoyl]succinic acid.

5. The compound according to claim 1, wherein the compound is $N^1,N^1,N^4,N^4$-tetrabutyldiamide of 2-[bis(4-dimethylaminophenyl)-phosphinoyl]succinic acid.

6. The compound according to claim 1, wherein the compound is $N^1,N^1,N^4,N^4$-tetrabutyldiamide of 2-[bis(4-chlorophenyl)-phosphinoyl]succinic acid

7. The compound according to claim 1, wherein the compound is $N^1,N^1,N^4,N^4$-tetrabutyldiamide of 2-[bis(4-methoxyphenyl)-phosphinoyl]succinic acid.

8. The compound according to claim 1, wherein the compound is $N^1,N^1,N^4,N^4$-tetrabutyldiamide of 2-(di-n-butylphosphinoyl)-succinic acid.

9. The compound according to claim 1, wherein the compound is $N^1,N^1,N^4,N^4$-tetrabutyldiamide of 2-[bis(4-methoxyphenyl)-phosphinoylmethyl]succinic acid.

10. The compound according to claim 1, wherein the compound is $N^1,N^1,N^4,N^4$-tetrabutyldiamide of 2-[bis(2,5-dimethylphenyl)-phosphinoylmethyl]succinic acid.

11. The compound according to claim 1, wherein the compound is $N^1,N^1,N^4,N^4$-tetraethyldiamide of 2-(diphenyl-phosphinoylmetyl)succinic acid.

12. A method of synthesizing the compounds according to claim 1 of general formula (I), comprising reacting phosphorylated 1,4-dicarboxylic acids of general formula (II),

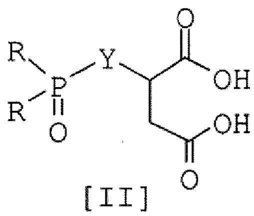

[II]

wherein R and Y have the meanings as defined in claim 1, with hexaalkyl triamidophosphites of general formula $(R'_2N)_3P$, wherein R' has the meanings as defined in claim 1, being heated in an organic solvent selected from

aromatic hydrocarbons or saturated hydrocarbons, **characterized in that** the reaction is conducted within a temperature range from 20 to 80°C.

## INTERNATIONAL SEARCH REPORT

International application No.
PCT/RU 2011/000843

A. CLASSIFICATION OF SUBJECT MATTER

C07F 9/53 (2006.01) A61K 31/66 (2006.01) A61P 25/28 (2006.01)

According to International Patent Classification (IPC) or to both national classification and IPC

B. FIELDS SEARCHED

Minimum documentation searched (classification system followed by classification symbols)

C07F 9/53, A61K 31/66, A61P 25/28

Documentation searched other than minimum documentation to the extent that such documents are included in the fields searched

Electronic data base consulted during the international search (name of data base and, where practicable, search terms used)

STN, RUPAT, EAPO, Esp@cenet, PAJ, USPTO, CIPO, DEPATIS, PCT Online

C. DOCUMENTS CONSIDERED TO BE RELEVANT

| Category* | Citation of document, with indication, where appropriate, of the relevant passages | Relevant to claim No. |
|---|---|---|
| X<br>A | CROWE, M. C. et al. Investigating bidentate and tridentate earbamoylmethylphosphine oxide ligand interactions with rare-earth elements using electrospray ionization quadrupole ion trap mass spectrometry. Inorganic Chemistry, 2005, 44(18), 6415-6424, especially the abstract, p. 6416, compound B | 1-2<br>3-12 |
| X<br>A | TURANOV A. N. et al. Ekstraktsiya U(VI), Th(IV), Pu(IV), Am(III) i redkozemelnykh elementov iz azotnokislykh rastvorov oksidami difenil (dialkilkarbamoilmetil) fosfinov s zamestitelyami v metilenovom mostike. Radiokhimiya, 2004, vol. 46, N° 5, p. 427-432, especially p. 428, compound IV and the example of the result | 1-2<br>3-12 |
| A | FRANK C. TORTELLA et al. ((Neuroprotection produced by the NAALADase inhibitor 2-PMPA in rat cerebellar neurons». European Journal of Pharmacology, 2000, v. 402, issues 1-2, pp. 31-37 (abstract) [on-line] [Found 2012-01-27] Found from the Internet: http ://www. sciencedirect.com/science/article/pii/S0014299900005197. | 1-11 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 26 January 2012 (26.01.2012) | 11 March 2012 (11.03.2012) |
| Name and mailing address of the ISA/<br><br>Facsimile No. | Authorized officer<br><br>Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 186950 A
- US 4588833 A
- US 3288794 A
- RU 2405789
- RU 2008129151
- RU 2413733
- RU 2008129150


### Non-patent literature cited in the description


- **B.O. KOZ'MINYKH.** Synthesis and biological activity of substituted amides and hydrazides of 1,4-dicarboxylic acids (a review). *Pharmaceutical Chemistry Journal (Rus),* vol. 40 (1), 9-17
- **A.N. TURANOV ; V.K. KARANDASHEV ; A.N. YARKEVICH ; Z. V. SAFRONOVA ; A.V. KHARITONOV ; N.I. RODYGINA ; A.M. FEDOSEEV.** Extraction of U(VI), Th(IV), Pu(IV), Am(III), and rare-earth elements from nitric acid solutions with diphenyl(dialkylcarbamoylmethyl)phosphine oxides substituted in the methylene bridge. *Radiochemistry,* 2004, vol. 46 (5), 427-432
- **HAJOS F.** *Brain Res.,* 1975, vol. 93 (3), 485-489
- **L.N. PETROVA ; S.O. BACHURIN.** *Bull. Exp. Biol. Med.,* 2006, vol. 142 (7), 51-54